# EUROPEAN PATENT SPECIFICATION

(11) **EP 1 732 638 B1**
(45) Date of publication and mention of the grant of the patent: **15.07.2009**
(21) Application number: 05729525.5
(22) Date of filing: 06.04.2005
(51) Int. Cl.: A61M 31/00, A61M 3/00

(54) **CANNULA FOR DISPENSING FLUID PRODUCTS FOR VAGINAL AND ANAL APPLICATIONS**
KANÜLE ZUR ABGABE VON FLÜSSIGEN PRODUKTEN FÜR VAGINALE UND ANALE ANWENDUNGEN
CANULE POUR LA DISTRIBUTION DE PRODUITS FLUIDES POUR APPLICATIONS VAGINALES ET ANALES

(30) Priority: 08.04.2004 IT MO20040073
(43) Date of publication of application: 20.12.2006
(73) Proprietor: LAMEPLAST S.p.A., 41030 Novi Di Modena, Frazione Rovereto Sul Secchia (IT)
(72) Inventor: FONTANA, Antonio, I-41012 CARPI (IT)
(74) Representative: Brogi, Graziano
(86) International application number: PCT/EP2005/051532
(87) International publication number: WO 2005/097251

(56) References cited:
- WO-A-02/083230
- WO-A-20/05030313
- CH-A- 199 616
- US-A- 5 045 058
- US-A- 5 292 307

## Description

The present invention relates to a cannula for dispensing fluid products for vaginal and anal applications, particularly for applying ointments, creams, medicinal pastes or the like. The features described in the preamble of claim 1 are known for example from CH-A-199 616.

### Background Art

Cannulas for dispensing medicinal fluid products are known which are used in particular for vaginal and anal applications and generally are sold in packages together with tubes or bottles that contain one of said products.

Known cannulas are constituted by a cylinder that is suitable to contain the product and inside which a plunger, rigidly coupled to the end of a pusher rod, can slide.

The cylinder has an open end, which can be coupled to the dispensing outlet of the tube, in order to introduce in said cylinder the amount of product to be applied, and through which the introduced product is dispensed.

The opposite end of the cylinder is closed by a bottom provided with a hole, in which the pusher rod is inserted so that it can slide and which acts as an element for stopping the plunger in order to prevent its extraction.

The product introduced in the cannula is dispensed by acting on the pusher rod in the direction of the sliding of the plunger toward the open end of the cylinder.

Cannulas are also known which are constituted by a cylinder, in which the opposite ends are open and inside which a plunger is inserted so that it can slide, and by a pusher rod, which is separate from the plunger and can be coupled detachably thereto.

Abutment undercuts or shoulders are formed at the opposite ends of the cylinder and are suitable to stop the sliding of the plunger, preventing it from disengaging under the action of the pusher rod.

These cannulas can be sold in packages that contain a single pusher rod, a plurality of empty single-use cylinders to be used for the various applications, and one or more tubes of product.

In this case, one of the two ends of the cylinders can be coupled to the dispensing outlet of the tube to introduce the product in said cylinders, while the opposite end acts as a passage for the pusher rod.

As an alternative, these cannulas can be sold in packages that contain a single pusher rod and a plurality of cylinders that are already filled with the product to be applied and are provided, at their two opposite ends, with closure plugs, which are removed at the time of use.

These known cannulas, however, are not free from drawbacks, including the fact that they have significant dimensions that make them difficult and awkward to handle for users.

Another drawback of known cannulas, particularly those constituted by a rod that is detachably associable with the plunger, is that they are not ready for immediate use by users, as they require additional assembly operations.

Another drawback of known cannulas is that the sliding of the plunger under the thrust applied to the rod often causes uncontrolled dispensing of amounts of product that are unwanted, since they are excessive or insufficient for the actual requirements of use.

### Disclosure of the Invention

The aim of the present invention is to eliminate the drawbacks noted above of known cannulas, by providing a cannula for dispensing fluid products for vaginal and anal applications that is compact, simple and straightforward to use for users, and allows controlled and gradual dispensing of the product.

Within this aim, an object of the present invention is to provide a structure that is simple, relatively easy to provide in practice, safe in use, effective in operation, and has a relatively low cost.

This aim and this and other objects that will become better apparent hereinafter are achieved by the present cannula for dispensing fluid products for vaginal and anal applications, comprising: an inner tubular body for containing a fluid product, which is provided, at a first end, with an outlet for dispensing said product and, at a second end that lies opposite the first end, with a handgrip; at least one straight longitudinal slot, which is formed on the lateral surface of said inner tubular body; an outer tubular body, which is open at its two opposite ends and is fitted substantially coaxially over said inner tubular body and is associated therewith with a rotary coupling; a helical guide, which is formed on the inner lateral surface of said outer tubular body; a plunger, which is accommodated in said inner tubular body, with which it is associated so that it can slide axially, and is provided with at least one pin, which is inserted so that it can slide in said straight longitudinal slot and has an end that extends beyond said straight longitudinal slot and mates with said helical guide, the relative rotation of said inner tubular body and of said outer tubular body being suitable to move said plunger so that it slides along said inner tubular body to expel said product from said dispensing outlet; a covering jacket, which is provided with an open end and a closed end and is fitted detachably on said outer tubular body; and tamper-resistant means for sealing said covering jacket to said handgrip, characterized in that said tamper-resistant sealing means comprise a plurality of bridges, which have an end that is associated with said covering jacket and an opposite end that is associated with a band that is rigidly coupled to said handgrip, breakable regions being formed proximate to at least one of the opposite ends of said bridges.

### Brief Description of the Drawings

Further characteristics and advantages of the present invention will become better apparent from the following detailed description of a preferred but not exclusive embodiment of a cannula for dispensing fluid products for vaginal and anal applications, illustrated by way of nonlimiting example in the accompanying drawings, wherein:
Figure 1 is a front view of the cannula according to the invention in the closed configuration for packaging;
Figure 2 is a front view of the cannula according to the invention in the open configuration for use;
Figure 3 is a schematic sectional view of the cannula according to the invention in the closed configuration for packaging;
Figure 4 is an enlarged-scale view of a detail of Figure 3;

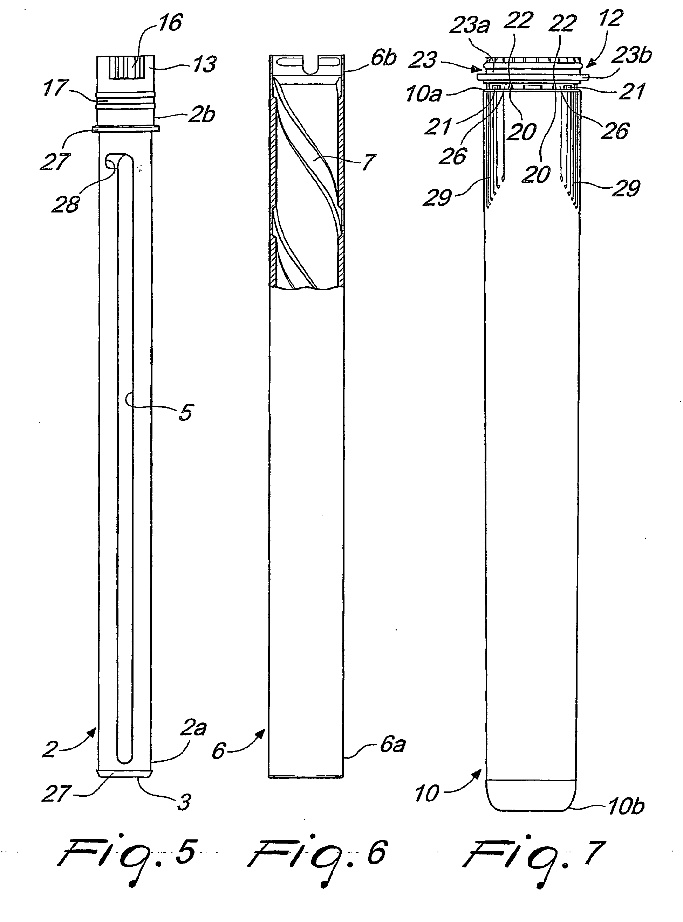

Figure 5 is a schematic view of the inner tubular body of the cannula according to the invention;
Figure 6 is a partially sectional schematic view of the outer tubular body of the cannula according to the invention;
Figure 7 is a schematic view of the covering jacket of the cannula according to the invention;

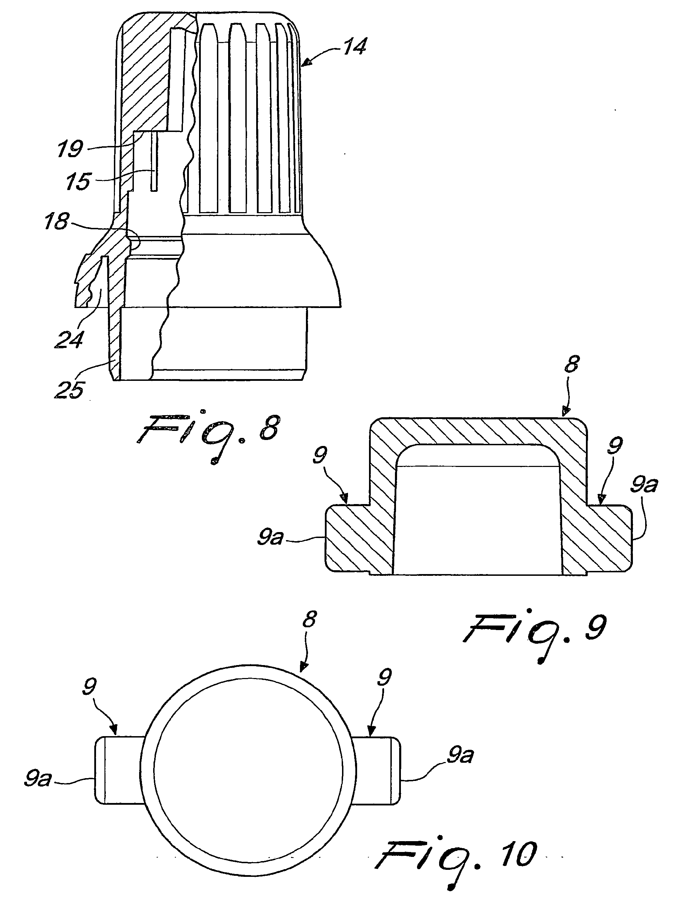

Figure 8 is a partially sectional schematic view of a detail of the handgrip of the cannula according to the invention;
Figure 9 is a schematic sectional view of the plunger of the cannula according to the invention;
Figure 10 is a front view of the plunger of the cannula according to the invention.

### Ways of causing out the Invention

With reference to the figures, the reference numeral 1 generally designates a cannula for dispensing fluid products P for vaginal and anal applications, particularly for the application of ointments, creams, medicinal pastes or the like.

The cannula 1 comprises an inner tubular body 2 for containing the product P, which has, at a first end 2a, an outlet 3 for dispensing the product P and, at a second end 2b arranged opposite the first end, a handgrip 4; at least one and preferably two diametrically opposite longitudinal straight slots 5 are provided on the lateral surface of the inner tubular body 2.

An outer tubular body 6 is fitted substantially coaxially over the inner tubular body 2, is open at the two opposite ends 6a and 6b, and is associated with the inner tubular body 2 with a rotary coupling; a groove-shaped helical guide 7 runs between the two opposite ends 6a and 6b and is provided on the inner lateral surface of the outer tubular body 6.

A plunger 8 is accommodated within the inner tubular body 2, with which it is associated so that it can slide axially; the plunger 8 is provided with at least one and preferably two mutually diametrically opposite pins 9, which are inserted so that they can slide in a respective slot 5 and have a respective end 9a that protrudes beyond the slots 5 in order to mate slidingly within the helical guide 7.

The cannula 1 further comprises a covering jacket 10 of the removable type, which is fitted over the outer tubular body 6; the jacket 10 is of the tubular type, with an open end 10a, for inserting and extracting the outer tubular body 6 in and from it, and an opposite end 10b which is closed and at which there is a sealing portion 11 having an inside diameter that is substantially smaller than the outside diameter of the end 6a of the outer tubular body 6.

When the cannula 1 is in the closed packaging configuration, the sealing portion 11 adheres to the end 6a, applying thereto a slight pressure, which is suitable to avoid seepages of product P between the jacket 10 and the outer tubular body 6.

The handgrip 4 and the jacket 10 are anchored temporarily to each other by tamper-resistant sealing means 12.

The handgrip 4 comprises a tang 13, which is formed at the second end 2b of the inner tubular body 2 and protrudes beyond the outer tubular body 6, and a cap 14, which is fitted rigidly over the tang 13.

Between the tang 13 and the cap 14 there are means for locking relative rotation and means for locking the relative axial sliding of one with respect to the other.

The means for locking the relative rotation between the tang 13 and the cap 14 can be of the type of a side-fit coupling or the like, and can be constituted for example by a plurality of knurled portions 15, which lie longitudinally on at least one portion of the inner lateral surface of the cap 14 and engage corresponding knurled portions 16 formed on a respective portion of the outer lateral surface of the tang 13; advantageously, the portions are two and are mutually diametrically opposite.

The means for locking the relative axial sliding between the tang 13 and the cap 14 can be of the interlocking type and be constituted for example by a groove 17, which is formed on the outer lateral surface of the tang 13 and mates by interlocking with a corresponding ring 18 formed on the inner lateral surface of the cap 14.

An abutment 19 for the tang 13 is also formed inside the cap 14.

The cap 14 is thus rigidly coupled to the tang 13 and therefore to the inner tubular body 2.

The tamper-resistant sealing means 12 comprise a plurality of bridges 20, which are distributed, so as to be alternated with spacers 21, along the perimetric rim of the open end 10a of the jacket 10 and have an end that is rigidly coupled to said rim and an opposite end that is coupled temporarily, at respective tearable/breakable regions 22, to a band 23 that is suitable to be rigidly coupled to the handgrip 4.

The band 23 comprises a fixing flap 23a, which is rigidly coupled to the perimetric rim of the grip 4, for example by interlocking in a seat 24 formed along the perimetric rim of the cap 14, and an indicator flap 23b, which protrudes from the handgrip 4 (seat 24) to indicate that the cannula 1 has been opened.

As an alternative, the fixing flap 23a could be rigidly coupled to the handgrip 4 with systems other than interlocking, for example by adhesive bonding, welding, ultrasound welding, or others.

The step for assembling the jacket 10 on the outer tubular body 6 occurs by pushing; in particular, the band 23, coupled to the bridges 20, is pushed in the seat 24; the spacers 21 act, during this step, as buffers, reducing the flexing of the bridges 20 and therefore the risk of their breakage.

The cap 14 is extended by a tubular sealing tab 25, which covers a preset extent inside the jacket 10 and is suitable to prevent air, dust or other external agents from entering the jacket 10 through the gaps 26 left free between the bridges 20 and the spacers 21.

Shoulders 27, respectively for the abutment of the opposite ends 6a and 6b of the outer tubular body 6, are formed at the first end 2a and at the second end 2b of the inner tubular body 2.

The stroke of the plunger 8 is limited by retention grooves 28 for the pins 9, which are formed at at least one of the opposite ends of the slots 5 and substantially transversely thereto.

Conveniently, on the outer lateral surface of the jacket 10 and of the outer tubular body 6 there are grip recesses 29 of the corrugated or knurled type, which are suitable to ensure the manual grip of a user, preventing the user's fingers from slipping during the maneuvers for using the cannula 1 and in particular the relative rotation between the handgrip 4 and the jacket 10 to open the cannula 1 and between the handgrip 4 (rigidly coupled to the inner tubular body 2) and the outer tubular body 6, for dispensing the product P.

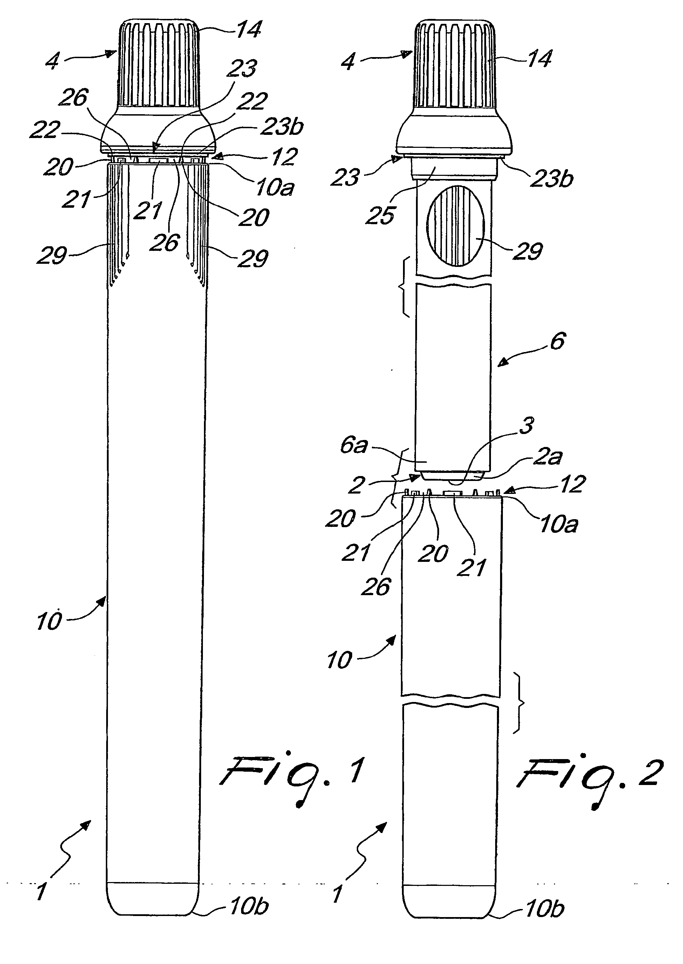

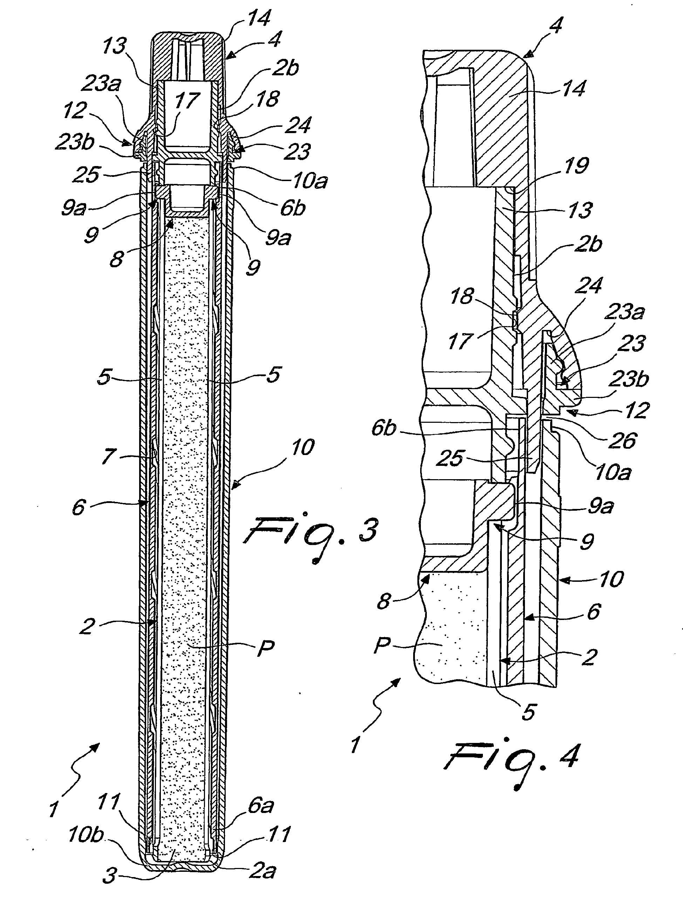

The cannula 1 is sold in the closed packaging configuration (Figures 1 and 3), filled with the product P; in this configuration, the jacket 10 is fitted over the outer tubular body 6 so as to close the dispensing outlet 3 and is anchored temporarily, by way of the tamper-resistant sealing means 12, to the handgrip 4, while the plunger 8 is arranged at the beginning of its stroke proximate to the tang 13.

To open the cannula 1, it is necessary to rotate the jacket 10 with respect to the handgrip 4, thus breaking the tearable/breakable regions 22 that temporarily couple the bridges 20 to the band 23, which is rigidly coupled to the handgrip 4; the jacket 10 is thus disengaged from the handle 4, the bridges 20 remain rigidly coupled to the jacket 10, and the strap 23 remains coupled to the cap 14 with the indicator flap 23b that protrudes therefrom to indicate that opening has occurred.

By extracting the jacket 10, the dispensing outlet 3 is connected to the outside and the cannula 1 is in the open configuration for use, ready for the application of the product P (Figure 2).

For application, it is sufficient to act on the handgrip 4, keeping motionless the outer tubular body 6, in order to turn the inner tubular body 2 with respect to the outer tubular body 6; as a consequence of said relative rotation, the resulting force that acts on the pins 9, locked so as to slide along the slots 5 and along the helical guide 7, moves the plunger 8 so that it slides axially along the inner tubular body 2 in order to expel the product P from the dispensing outlet 3.

In practice it has been found that the described invention achieves the proposed aim and object.

The cannula according to the invention in fact is compact, particularly with respect to known cannulas provided with a pusher rod, making it easier to handle.

Moreover, the cannula according to the invention is simple and straightforward to use and, by way of the pressure applied to the plunger following the relative rotation imparted by the user to the inner tubular body with respect to the outer tubular body, allows better control and graduality of the dispensing of the product packaged therein.

The invention thus conceived is susceptible of numerous modifications and variations, all of which are within the scope of the appended claims.

All the details may further be replaced with other technically equivalent ones.

In practice, the materials used, as well as the shapes and the dimensions, may be any according to requirements without thereby abandoning the scope of the protection of the appended claims.

## Claims

1. A cannula for dispensing fluid products for vaginal and anal applications, comprising: an inner tubular body (2) for containing a fluid product, which is provided, at a first end, with an outlet (3) for dispensing said product and, at a second end that lies opposite the first end, with a handgrip (4), at least one straight longitudinal slot (5), which is formed on the lateral surface of said inner tubular body; an outer tubular body (6), which is open at its two opposite ends and is fitted substantially coaxially over said inner tubular body and is associated therewith with a rotary coupling; a helical guide (7), which is formed on the inner lateral surface of said outer tubular body; a plunger (8), which is accommodated in said inner tubular body, with which it is associated so that it can slide axially, and is provided with at least one pin (9), which is inserted so that it can slide in said straight longitudinal slot and has an end that extends beyond said straight longitudinal slot and mates with said helical guide, the relative rotation of said inner tubular body and of said outer tubular body being suitable to move said plunger so that it slides along said inner tubular body to expel said product from said dispensing outlet; a covering jacket (10), which is provided with an open end and a closed end and is fitted detachably on said outer tubular body; and tamper-resistant means (12) for sealing said covering jacket to said handgrip, **characterized in that** said tamper-resistant sealing means comprise a plurality of bridges (20), which have an end that is associated with said covering jacket and an opposite end that is associated with a band that is rigidly coupled to said handgrip, breakable regions (22) being formed proximate to at least one of the opposite ends of said bridges.

2. The cannula according to claim 1, **characterized in that** said bridges are distributed along the perimetric rim of said open end of the covering jacket.

3. The cannula according to one or more of the preceding claims, **characterized in that** said breakable regions are formed proximate to the end of said bridges that is associated with said band.

4. The cannula according to one or more of the preceding claims, **characterized in that** said tamper-resistant sealing means comprise a plurality of spacers, which are distributed along said perimetric rim of the open end of the covering jacket and are alternated with said bridges.

5. The cannula according to one or more of the preceding claims, **characterized in that** said handgrip comprises a tang, which is formed at said second end of the inner tubular body and extends beyond said outer tubular body, and a cap, which is fitted rigidly over said tang, between said tang and said cap there being means for locking the relative rotation and means for locking the relative sliding of one with respect to the other.

6. The cannula according to claim 5, **characterized in that** said means for locking the relative rotation of said tang and said cap are of the type of a side-fit coupling or the like.

7. The cannula according to claims 5 or 6, **characterized in that** said means for locking the relative rotation of said tang and said cap comprise a plurality of knurled portions, which run longitudinally along at least one portion of the inner lateral surface of said cap and mesh with corresponding knurled portions formed on a respective portion of the outer lateral surface of said tang.

8. The cannula according to one or more of claims 5 to 7, **characterized in that** said means for locking the relative sliding of said tang and said cap are of the interlocking type.

9. The cannula according to one or more of claims 5 to 8, **characterized in that** said means for locking the relative sliding of said tang and said cap comprise at least one ring, which protrudes from said inner lateral surface of said cap and mates with an interlocking coupling with a corresponding annular groove formed on the outer lateral surface of said tang.

10. The cannula according to one or more of the preceding claims, **characterized in that** said band comprises a fixing flap, which is rigidly coupled to the perimetric rim of said cap, and an indicator flap, which protrudes from said cap.

11. The cannula according to one or more of the preceding claims, **characterized in that** a sealing portion is formed proximate to said closed end of said covering jacket and has an inside diameter that is substantially smaller than the outside diameter of said outer tubular body.

12. The cannula according to one or more of the preceding claims, **characterized in that** said cap is extended by a tubular sealing tab, which lies for a preset extent within said covering jacket and is suitable to prevent the penetration therein of external agents through the free gaps between said bridges and/or said spacers.

13. The cannula according to one or more of the preceding claims, **characterized in that** said first and second ends of the inner tubular body comprise abutment shoulders respectively for the opposite ends of said outer tubular body.

14. The cannula according to one or more of the preceding claims, **characterized in that** said straight longitudinal slots are two and are diametrically mutually opposite, said piston being provided with respective two of said pins, which are mutually diametrically opposite.

15. The cannula according to one or more of the preceding claims, **characterized in that** it comprises grooves for stopping the stroke of said plunger, which are formed at at least one of the opposite ends of said straight longitudinal slots in a direction that is substantially transverse thereto.

16. The cannula according to one or more of the preceding claims, **characterized in that** said helical guide is formed by a groove that lies between the opposite ends of said outer tubular body.

17. The cannula according to one or more of the preceding claims, **characterized in that** it comprises grip impressions, of the type with a corrugated or knurled surface or the like, which are formed on the outer lateral surface of said covering jacket and/or of said outer tubular body.

## Patentansprüche

1. Kanüle zur Abgabe von flüssigen Produkten für vaginale und anale Anwendungen mit:
einem röhrenförmigen Innenkörper (2) zum Aufbewahren eines flüssigen Produktes, der - an einem ersten Ende - mit einem Auslass (3) zur Abgabe des Produktes bereitgestellt ist, und - an einem zweiten Ende, welches dem ersten Ende gegenüberliegt - mit einem Handgriff (4) versehen ist; mindestens einem gerade verlaufenden Längsschlitz (5), der mit der seitlichen Fläche des röhrenförmigen Innenkörpers ausgebildet wird; einem röhrenförmigen Außenkörper (6), der an seinen beiden sich gegenüberliegenden Enden geöffnet ist und im Wesentlichen koaxial über den röhrenförmigen Innenkörper eingepasst wird, wobei er mit diesem in einer Drehverkopplung verbunden ist; einer Schrägverzahnungsführung (7), die in der seitlichen Innenfläche des röhrenförmigen Außenkörpers ausgebildet ist; einem Plungerkolben (8), der in dem röhrenförmigen Innenkörper aufgenommen ist, wobei er so angeschlossen ist, dass er sich axial verschieben kann, und mit mindestens einem Kolbenbolzen (9) bereitgestellt ist, der so eingesetzt ist, dass er in dem gerade verlaufenden Längsschlitz gleiten kann, und der ein Ende besitzt, das sich über den geraden Längsschlitz hinaus verlängert und mit der Schrägverzahnungsführung in Eingriff steht, wobei ein relatives Drehen des röhrenförmigen Innenkörpers und des röhrenförmigen Außenkörpers dient, den Kolben zu verschieben, so dass er entlang des röhrenförmigen Innenkörpers gleitet, um das Produkt aus der Spenderausgabe auszustoßen; einer verkleidenden Ummantelung (10), die mit einem geöffneten und einem geschlossenen Ende bereitgestellt und auf dem röhrenförmigen Außenkörper abnehmbar eingepasst ist; und einer manipulationssicheren Einrichtung (12) zur Abschirmung der Ummantelung in Bezug auf den Handgriff, **gekennzeichnet dadurch, dass** die manipulationssichere Abschirmeinrichtung eine Vielzahl von Stegübergängen (20) aufweist, die ein Ende besitzen, das mit der Ummantelung verbunden ist und ein gegenüberliegendes Ende, das mit einem Bundring verbunden ist, welcher mit dem Handgriff starr verkoppelt ist, sowie durch brechbare Bereiche (22), die unmittelbar zu mindestens einem der gegenüberliegenden Enden der Übergänge ausgebildet sind.

2. Kanüle gemäß Anspruch 1, **dadurch gekennzeichnet, dass** die Stegübergänge rund um den Perimeterrand an dem geöffneten Ende der verkleidenden. Ummantelung verteilt sind.

3. Kanüle gemäß einem oder mehreren der vorhergehenden Ansprüche, **dadurch gekennzeichnet, dass** die brechbaren Bereiche unmittelbar an dem Ende der Übergänge ausgebildet sind, welches mit dem Bundring in Verbindung steht.

4. Kanüle gemäß einem oder mehreren der vorhergehenden Absprüche, **dadurch gekennzeichnet, dass** die manipulationssicheren Abschirmeinrichtungen eine Vielzahl von Abstandshaltern aufweisen, die rund um den Perimeterrand des geöffneten Endes der Ummantelung verteilt sind und sich mit den Stegübergängen abwechseln.

5. Kanüle gemäß einem oder mehreren der vorhergehenden Absprüche, **dadurch gekennzeichnet, dass** der Handgriff einen Mitnehmer umfasst, der am zweiten Ende des röhrenförmigen Innenkörpers ausgebildet ist und sich über den röhrenförmigen Außenkörper erstreckt, und eine Kappe aufweist, die über dem Mitnehmer starr eingepasst ist, wobei zwischen Kappe und Mitnehmer Mittel zum Arretieren der relativen Drehung und Mittel zum Arretieren der relativen Verschiebung - des einen in Bezug auf das andere - vorhanden sind.

6. Kanüle gemäß Anspruch 5, **dadurch gekennzeichnet, dass** die Mittel zum Arretieren der relativen Drehung des Mitnehmers und der Kappe ein Seiteneinpass-Verkopplungstyp oder desgleichen Typs sind.

7. Kanüle gemäß Anspruch 5 oder 6, **dadurch gekennzeichnet, dass** die Mittel zum Arretieren der relativen Drehung des Mitnehmers und der Kappe eine Vielzahl von gerändelten Abschnitten aufweisen, die longitudinal entlang mindestens eines Abschnittes in der seitlichen Innenfläche der Kappe verlaufen und mit korrespondierenden, gerändelten Abschnitten ineinander greifen, die auf einem entsprechenden Abschnitt der seitlichen Außenfläche des Mitnehmers ausgebildet sind.

8. Kanüle gemäß einem oder mehreren der Ansprüche 5 bis 7, **dadurch gekennzeichnet, dass** die Mittel zum Arretieren der relativen Verschiebung des Mitnehmers und der Kappe ein Verzahnungstyp sind.

9. Kanüle gemäß einem oder mehreren der Ansprüche 5 bis 8, **dadurch gekennzeichnet, dass** die Mittel zum Arretieren der relative Verschiebung des Mitnehmers und der Kappe mindestens einen Ring aufweisen, welcher von der seitlichen Innenfläche der Kappe hervorragt und mit einer Verzahnungskopplung in eine korrespondierende, ringförmige Nut ineinandergreift, die an der seitlichen Außenfläche des Mitnehmers ausgebildet ist.

10. Kanüle gemäß einem oder mehreren der vorhergehenden Ansprüche, **dadurch gekennzeichnet, dass** der Bundring eine Befestigungsklappe aufweist, die mit dem Perimeterrand der Kappe starr verbunden ist, und eine Indikatorklappe umfasst, die von der Kappe hervorsteht.

11. Kanüle gemäß einem oder mehreren der vorhergehenden Ansprüche, **dadurch gekennzeichnet, dass** ein Dichtungsabschnitt unmittelbar an dem geschlossenen Ende der Ummantelung ausgebildet ist, und einen Innendurchmesser besitzt, der wesentlich kleiner als der Außendurchmesser des röhrenförmigen Außenkörpers ist.

12. Kanüle gemäß einem oder mehreren der vorhergehenden Ansprüche, **dadurch gekennzeichnet, dass** sich die Kappe mithilfe einer röhrenförmigen Dichtungslasche verlängert, die sich in einem vorher festgelegten Größenumfang innerhalb der Ummantelung befindet und dazu dient, das Eindringen von externen Stoffen durch die freien Lücken zwischen den Stegübergängen und/oder den Abstandshaltern zu verhindern.

13. Kanüle gemäß einem oder mehreren der vorhergehenden Ansprüche, **dadurch gekennzeichnet, dass** das erste und das zweite Ende des röhrenförmigen Innenkörpers in Bezug auf die gegenüberliegenden Enden des röhrenförmigen Außenkörpers jeweils Anschlagvorsprünge aufweisen.

14. Kanüle gemäß einem oder mehreren der vorhergehenden Ansprüche, **dadurch gekennzeichnet, dass** die gerade verlaufenden Längsschlitze zwei sind und zueinander entgegengesetzt angeordnet sind, wobei der Kolben jeweils mit den beiden Kolbenbolzen bereitgestellt wird, die zueinander entgegengesetzt angeordnet sind.

15. Kanüle gemäß einem oder mehreren der vorhergehenden Ansprüche, **dadurch gekennzeichnet, dass** sie Gewinderillen zum Stoppen des Hubs des Plungerkolbens aufweist, welche mindestens auf einem der sich gegenüberliegenden Enden der geraden Längsschlitze ausgebildet sind, und zwar in eine Richtung, die zu diesen im Wesentlichen quer verlaufend angeordnet sind.

16. Kanüle gemäß einem oder mehreren der vorergehenden Ansprüche, **dadurch gekennzeichnet, dass** die Schrägverzahnungsführung mittels einer Gewinderille ausgebildet ist, die sich zwischen den sich gegenüberliegenden Enden des röhrenförmigen Außenkörpers befindet.

17. Kanüle gemäß einem oder mehreren der vorhergehenden. Ansprüche, **dadurch gekennzeichnet, dass** sie Griffprägungen aufweist, die flächenmäßig gewellt oder gerändelt oder desgleichen Typs sind, die auf der seitlichen Außenfläche der verkleidenden Ummantelung und/oder auf dem röhrenförmigen Außenkörper ausgebindet sind.

## Revendications

1. Canule pour délivrer des produits fluides destinés à des applications vaginales et anales, comprenant : un corps tubulaire intérieur (2) destiné à contenir un produit fluide, qui est pourvu, à une première extrémité, d'une ouverture (3) pour distribuer ledit produit et, à une deuxième extrémités qui est opposée à la première extrémité, d'un manche (4), d'au moins une fente longitudinale droite (5) qui est formée sur la surface latérale dudit corps tubulaire intérieur ; un corps tubulaire extérieur (6) qui est ouvert à ses deux extrémités opposées et est placé essentiellement coaxialement sur ledit corps tubulaire intérieur et est associé à lui avec un accouplement rotatif ; un guide hélicoïdal (7) qui est formé sur la surface latérale intérieure dudit corps tubulaire extérieur; un piston (8), qui est reçu dans ledit corps tubulaire intérieur avec lequel il est associé de manière à pouvoir coulisser axialement, et est pourvu d'au moins un ergot (9) qui est inséré de manière à pouvoir coulisser dans ladite fente longitudinale droite et possède une extrémité qui s'étend au-delà de ladite fente longitudinale droite et s'accouple avec ledit guide hélicoïdal, la rotation relative dudit corps tubulaire intérieur et dudit corps tubulaire extérieur étant apte à déplacer ledit piston de façon qu'il coulisse le long dudit corps tubulaire intérieur de manière à expulser ledit produit par ladite ouverture de distribution ; une gaine de couverture (10) qui est pourvue d'une extrémité ouverte et d'une extrémité fermée et est placée de manière détachable sur ledit corps tubulaire extérieur; et des moyens de fermeture inviolable (12) destinés à fixer ladite gaine de couverture audit manche, ***caractérisée en ce que*** lesdits moyens de fixation à fermeture inviolable comprennent une pluralité de pontets (20) qui ont une extrémité qui est associée à ladite gaine de couverture et une extrémité opposée qui est associée à une bande qui est couplée de manière rigide audit manche, des zones ruptibles (22) étant formées à proximité d'au moins une des extrémités opposées desdits pontets.

2. Canule selon la revendication 1, ***caractérisée en ce que*** lesdits pontets sont répartis le long du bord périmétrique de ladite extrémité ouverte de la gaine de couverture.

3. Canule selon l'une ou plusieurs des revendications précédentes, ***caractérisée en ce que*** lesdites zones ruptibles sont formées à proximité de l'extrémité desdits pontets qui est associée à ladite bande.

4. Canule selon l'une ou plusieurs des revendications précédentes, ***caractérisée en ce que*** lesdits moyens de fixation à fermeture inviolable comprennent une pluralité d'éléments d'espacement qui sont répartis le long dudit bord périmétrique de l'extrémité ouverte de la gaine de couverture et alternent avec lesdits pontets.

5. Canule selon l'une ou plusieurs des revendications précédentes, ***caractérisée en ce que*** ledit manche comprend une soie, qui est formée au niveau de ladite deuxième extrémité du corps tubulaire intérieur et s'étend au-delà dudit corps tubulaire extérieur, et un capuchon, qui est fixé rigidement sur ladite soie, des moyens pour bloquer la rotation relative et des moyens pour bloquer le glissement relatif les uns par rapport aux autres étant prévus entre ladite soie et ledit capuchon.

6. Canule selon la revendication 5, ***caractérisée en ce que*** lesdits moyens pour bloquer la rotation relative de ladite soie et dudit capuchon sont du type couplage à ajustement latéral ou similaire.

7. Canule selon les revendications 5 ou 6, ***caractérisée en ce que*** lesdits moyens pour bloquer la rotation relative de ladite soie et dudit capuchon comprennent une pluralité de portions moletées qui courent longitudinalement le long d'au moins une partie de la surface latérale intérieure dudit capuchon et s'emboîtent avec des portions moletées correspondantes formées sur une portion respective de la surface latérale extérieure de ladite soie.

8. Canule selon les revendications 5 à 7, ***caractérisée en ce que*** lesdits moyens pour bloquer le glissement relatif de ladite soie et dudit capuchon sont du type verrouillage réciproque.

9. Canule selon l'une ou plusieurs des revendications 5 à 8, ***caractérisée en ce que*** lesdits moyens pour bloquer le glissement relatif de ladite soie et dudit capuchon comprennent au moins une bague, qui fait saillie de ladite surface latérale intérieure dudit capuchon et s'accouple, avec un accouplement à verrouillage réciproque, avec une gorge annulaire correspondante formée sur la surface latérale extérieure de ladite soie.

10. Canule selon l'une ou plusieurs des revendications précédentes, ***caractérisée en ce que*** ladite bande comprend un rabat de fixation, qui est couplé de manière rigide au rebord périmétrique dudit capuchon, et un rabat indicateur, qui saille dudit capuchon.

11. Canule selon l'une ou plusieurs des revendications précédentes, ***caractérisée en ce* qu'**une portion de fermeture étanche est formée à proximité de ladite extrémité fermée de ladite gaine de ouverture et possède un diamètre intérieur qui est sensiblement inférieur au diamètre extérieur dudit corps tubulaire extérieur.

12. Canule selon l'une ou plusieurs des revendications précédentes, ***caractérisée en ce que*** ledit capuchon est prolongé par une languette d'étanchéité tubulaire, qui s'étend d'une longueur donnée dans ladite gaine de couverture et est apte à y empêcher la pénétration d'agents externes par des interstices libres entre lesdits pontets et/ou lesdits éléments d'espacement.

13. Canule selon l'une ou plusieurs des revendications précédentes, ***caractérisée en ce que*** lesdites première et deuxième extrémités du corps tubulaire intérieur comprennent des épaulements de butée respectivement pour les extrémités opposées dudit corps tubulaire extérieur.

14. Canule selon l'une ou plusieurs des revendications précédentes, ***caractérisée en ce que*** lesdites fentes longitudinales droites sont au nombre de deux et sont diamétralement opposées, ledit piston étant pourvu de deux, respectifs, desdits ergots, qui sont diamétralement opposés.

15. Canule selon l'une ou plusieurs des revendications précédentes, ***caractérisée en ce qu'***elle comprend des gorges pour arrêter la course dudit piston, qui sont formées sur au moins l'une des extrémités opposées desdites fentes longitudinales droites, dans une direction qui est sensiblement transversale à celles-ci.

16. Canule selon l'une ou plusieurs des revendications précédentes, ***caractérisée en ce que*** ledit guide hélicoïdal est formé par une gorge qui est ménagée entre les extrémités opposées dudit corps tubulaire extérieur.

17. Canule selon l'une ou plusieurs des revendications précédentes, ***caractérisée en ce* qu'**elle comprend des empreintes pour faciliter la prise, du type à surface ondulée ou moletée ou similaire, qui sont formées sur la surface latérale extérieure de ladite gaine de couverture et/ou dudit corps tubulaire extérieur.
